Europäisches Patentamt

European Patent Office

Office européen des brevets

(11) Publication number: **0 341 734**
**A2**

## (12) EUROPEAN PATENT APPLICATION

(21) Application number: 89108595.3

(22) Date of filing: 12.05.89

(51) Int. Cl.4: **C07K 15/04 , C07K 3/20 ,**
**A61K 37/02 , A61K 35/68 ,**
**//C07K7/10**

(30) Priority: 13.05.88 JP 117887/88

(43) Date of publication of application:
15.11.89 Bulletin 89/46

(84) Designated Contracting States:
AT BE CH DE ES FR GB GR IT LI LU NL SE

(71) Applicant: SUMITOMO PHARMACEUTICALS
COMPANY, LIMITED
2-8, Dosho-Machi 2-Chome, Chuo-Ku
Osaka-Shi Osaka-Fu(JP)

(72) Inventor: Suzuki, Naoyoshi
26-2, Yayoi-cho 3-chome
Nakano-ku Tokyo(JP)
Inventor: Yoshida, Yutaka
7-4, Kitazono 2-chome
Hirosaki Aomori(JP)
Inventor: Osaki, Humio
5-18, Zuigaoka Tarumi-ku
Kobe Hyogo(JP)
Inventor: Kojima, Shinichi
247-20, Azashounomoto Mikagechogunge
Higashinada-ku Kobe Hyogo(JP)
Inventor: Yanagi, Yoshikazu
5-4, Uguisudai 2-chome
Kawanishi Hyogo(JP)
Inventor: Suwa, Kazushi
15-10-303, Kusu-machi
Ashiya Hyogo(JP)

(74) Representative: Vossius & Partner
Siebertstrasse 4 P.O. Box 86 07 67
D-8000 München 86(DE)

(54) Proteins having anti-tumor activity.

(57) Physiologically active proteins having the following properties:
(1) having a molecular weight (determined by gel filtration with Sephadex G-75) of 4,000 to 15,000;
(2) obtainable from the water-soluble fraction of protozoan Toxoplasma;
(3) showing an anti-tumor activity against sarcoma transplanted on mouse.

The above proteins can be purified with a high efficiency by reverse phase liquid chromatography. Further, the purified proteins are useful as anti-tumor agents.

## PROTEINS HAVING ANTI-TUMOR ACTIVITY

The present invention relates to proteins having anti-tumor activity. More particularly, it relates to novel proteins producing an anti-tumor effect, which are obtainable from the tachyzoites of Toxoplasma gondii, a protozoan parasite , (hereinafter referred to as "Tp").

As physiologically active proteins obtainable from protozoan Toxoplasma, there is known one which is useful as an interferon-inducing agent (cf. JP-A-59-27832) and one which is useful as an anti-tumor agent (cf. JP-A-62-249927). When the physical destruction product of protozoan Toxoplasma is centrifuged at 10,000 G or higher and the resultant precipitate and supernatant are subjected to molecular weight fractionation by gel filtration, there are obtained water-soluble fractions having a molecular weight of more than 100,000, having a molecular weight between 20,000 and 100,000 and having a molecular weight of not more than 4,000, from which proteins exerting an interferon-inducing activity are obtainable. Proteins exerting an anti-tumor activity comprise the one having a molecular weight of 15,000 to 20,000 as the major component.

As a result of the extensive study on physiologically active substances in the water-soluble material of the physical destruction product of protozoan Toxoplasma, it has been found that proteins comprising the one having a molecular weight of 4,000 to 15,000 as the major component show an excellent anti-tumor activity. It has also been found that such proteins can be purified efficiently by the use of reverse phase liquid chromatography. This invention is based on these findings.

Accordingly, a main object of this invention is to provide proteins having an anti-tumor activity, which are obtainable from protozoan Toxoplasma. Another object of the invention is to provide a process for purification of said proteins. A further object of the invention is to provide an anti-tumor agent comprising said proteins.

The proteins according to this invention are characteristic in having the following properties:

(1) Having a molecular weight (determined by gel filtration with Sephadex G-75) of 4,000 to 15,000;

(2) Obtainable from the water-soluble fraction of protozoan Toxoplasma;

(3) Showing an anti-tumor activity against sarcoma transplanted on mouse.

The above proteins can be purified with a high efficiency by reverse phase liquid chromatography. Further, the purified proteins are useful as anti-tumor agents.

In the following descriptions, amino acids, protective groups, active groups, solvents, etc. are occasionally represented by abbreviations according to the IUPAC-IUB nomenclature or as conventionally used in the related art field. For instance, amino acid residues are represented by the following abbreviations: Arg, L-arginine; Leu, L-leucine; Val, L-valine; His, L-histidine; Thr, L-threonine; Ser, L-serine; Glu, L-Glutamic acid; Lys, L-lysine; Gln, L-Glutamine; Ile, L-isoleucine; Asn, L-asparagine; Tyr, L-tyrosine; Asp, L-aspartic acid; Gly, Glycine; Ala, L-alanine; Phe, L-phenylalanine; Pro, L-proline; Met, L-methionine; Asx, L-aspartic acid or L-asparagine; Glx, L-Glutamic acid or L-Glutamine.

Amino acid residues without the prefix "L" represent those having a naturally occuring absolute steric configuration unless otherwise indicated.

Other abbreviatons have the following meanings: Boc, t-butyloxycarbonyl; Cl-Z, 2-chlorobenzyloxycarbonyl; Tos, p-toluenesulfonyl; OcHex, cyclohexyl ester; Bzl, benzyl; Clz-Bzl, 2,6-dichlorobenzyl; TFA, trifluoroacetic acid; DCC, dicyclohexylcarbodiimide; DMF, dimethylformamide.

The anti-tumor proteins of the invention may comprise a single protein or two or more proteins in mixture insofar as the properties (1) to (3) are satisfied. Some typical examples of the anti-tumor proteins according to the invention are shown below.

TLA-H4

1) Color and appearance: white powder.

2) Water-solubility: easily soluble.

3) Molecular weight:

3-1) About 10,500 measured by high speed gel filtration using TSK-G2000SW. The marker proteins were bovine serum albumin (molecular weight, 67,000), egg albumin (molecular weight, 43,000), ribonuclease A (molecular weight, 13,700) and pork insulin (molecular weight, 5,800) manufactured by Sigma, and the eluting buffer was M/15 phosphate buffer-M/10 sodium chloride (pH, 6.7).

3-2) About 8,500 measured by SDS-polyacrylamide gel electrophoresis under non-reduction. The marker proteins were phosphorylase B (molecular weight, 94,000), bovine serum albumin (molecular weight, 67,000), egg albumin (molecular weight, 43,000), carbonic anhydrase (molecular weight, 30,000), soybean trypsin inhibitor (molecular weight, 21,000), lysozyme (molecular weight, 14,400) and pork insulin (molecular weight, 5,800), among which the pork insulin is the product by Sigma and all others are the products by Pharmacia.

4) N-Terminal amino acid sequence: Met-Gln-Ile-Phe-Val-Lys-Thr-Leu-Thr-Gly-Lys-Thr-Ile-.
The specimen was subjected to Edman's degradation using a 470A-type protein sequencer (Applied Biosystems Inc.), and the produced phenylthiohydantoin (PTH)-amino acid derivative was identified by a high speed liquid chromatography (manufactured by Shimadzu).

5) Amino acid composition:
Constant boiling point hydrochloric acid (0.2 ml) containing 0.1 % thioglycolic acid was added to TLA-H4 (1 $\mu$g), and the resultant mixture was hydrolyzed in a sealed tube at 110°C for 24 hours to determine the amino acid composition. The results are shown in Table 1.


TLA-H5A

1) Color and appearance: white powder
2) Water-solubility: easily soluble
3) Molecular weight:
3-1) About 10,500 measured by high speed gel filtration using TSK-G2000SW. The marker proteins were the same as used in regard to TLA-H4.
3-2) About 8,500 measured by SDS-polyacrylamide gel electrophoresis under non-reduction condition. The marker proteins were the same as used in regard to TLA-H4.

4) Amino acid sequence:

```
       1                                          10
       Met-Gln-Ile-Phe-Val-Lys-Thr-Leu-Thr-Gly-
                                                 20
       Lys-Thr-Ile-Thr-Leu-Asp-Val-Glu-Pro-Ser-
                                                 30
       Asp-Thr-Ile-Glu-Asn-Val-Lys-Ala-Lys-Ile-
                                                 40
       Gln-Asp-Lys-Glu-Gly-Ile-Pro-Pro-Asp-Gln-
                                                 50
       Gln-Arg-Leu-Ile-Phe-Ala-Gly-Lys-Gln-Leu-
                                                 60
       Glu-Asp-Gly-Arg-Thr-Leu-Ser-Asp-Tyr-Asn-
                                                 70
       Ile-Gln-Lys-Glu-Ser-Thr-Leu-His-Leu-Val-
                         74
       Leu-Arg-Leu-Arg
```

5) Amino acid composition:
Constant boiling point hydrochloric acid (0.2 ml) containing 0.1 % thioglycolic acid was added to TLA-H5A (2 $\mu$g), and the resultant mixture was hydrolyzed in a sealed tube at 110°C for 24 hours to determine the amino acid composition. The results are shown in Table 1.


TLA-H5C

1) Color and appearance: white powder.
2) Water-solubility: easily soluble.
3) Molecular weight:

3-1) About 10,500 measured by high speed gel filtration using TSK-G2000SW. The marker proteins were the same as used in regard to TLA-H4.

3-2) About 8,500 measured by SDS-polyacrylamide gel electrophoresis under non-reduction. The marker proteins were the same as used in regard to TLA-H4.

4) Amino acid sequence:

```
1                                              10
Met-Gln-Ile-Phe-Val-Lys-Thr-Leu-Thr-Gly-
                                               20
Lys-Thr-Ile-Thr-leu- X -Val-Glu-Pro-Ser-
                                               30
Asp-Thr-Ile-Glu-Asn-Val-Lys-Ala-Lys-Ile-
                                               40
Gln-Asp-Lys-Glu-Gly-Ile-Pro-Pro-Asp-Gln-
                                               50
Gln-Arg-Leu-Ile-Phe-Ala-Gly-Lys-Gln-Leu-
                                               60
Glu-Asp-Gly-Arg-Thr-Leu-Ser-Asp-Tyr-Asn-
                                               70
Ile-Gln-Lys-Glu-Ser-Thr-Leu-His-Leu-Val-
                    74
Leu-Arg-Leu-Glx
```

wherein X represents Asp or Glu.

5) Amino acid composition:
Constant boiling point hydrochloric acid (0.2 ml) containing 0.1 % thioglycolic acid was added to TLA-H5C (2 μg), and the resultant mixture was hydrolyzed in a sealed tube at 110°C for 24 hours to determine the amino acid composition. The results are shown in Table 1.

TLA-H6

1) Color and appearance: white powder.
2) Water-solubility: easily soluble.
3) Molecular weight:
3-1) About 10,500 measured by high speed gel filtration using TSK-G2000SW. The marker proteins were the same used in regard to TLA-H4.
3-2) About 8,500 measured by SDS-polyacrylamide gel electrophoresis under non-reduction. The marker proteins were the same as used in regard to TLA-H4.

4) Amino acid sequence:

```
1                                        10
Met-Gln-Ile-Phe-Val-Lys-Thr-Leu-Thr-Gly-
                                         20
Lys-Thr-Ile-Thr-leu- X -Val-Glu-Pro-Ser-
                                         30
Asp-Thr-Ile-Glu-Asn-Val-Lys-Ala-Lys-Ile-
                                         40
Gln-Asp-Lys-Glu-Gly-Ile-Pro-Pro-Asp-Gln-
                                         50
Gln-Arg-Leu-Ile-Phe-Ala-Gly-Lys-Gln-Leu-
                                         60
Glu-Asp-Gly-Arg-Thr-Leu-Ser-Asp-Tyr-Asn-
                                         70
Ile-Gln-Lys-Glu-Ser-Thr-Leu-His-Leu-Val-
                     74
Leu-Arg-Leu-Glx
```

wherein X represents Asp or Glu.

Still, TLA-H6 covers the one wherein X is Asp (hereinafter referred to as "TLA-H6D"), the one wherein X is Glu (hereinafter referred to as "TLA-H6E") and a mixture of them. TLA-H6 denotes a mixture of TLA-H6D and TLA-H6E, unless otherwise indicated.

5) Amino acid composition:

Constant boiling point hydrochloric acid (0.2 ml) containing 0.1 % thioglycolic acid was added to TLA-H6 (2 $\mu$g), and the resultant mixture was hydrolyzed in a sealed tube at 110°C for 24 hours to determine the amino acid composition. The results are shown in Table 1.

TLA-H8

1) Color and appearance: white powder.

2) Water-solubility: easily soluble.

3) Molecular weight:

About 14,000 measured by SDS-polyacrylamide gel electrophoresis under non-reduction. The marker proteins were the same as used in regard to TLA-H4.

4) N-Terminal amino acid sequence:

```
1                  5                  10
Lys-Val-Tyr-Glu-Arg- X - Glu-Phe-Ala-Arg-
                    15                 20
Thr-Leu-Lys-Arg-Asn-Gly-Met-Ala-Gly-Try-
                    25        27
Tyr-Gly-Val-Ser-Leu-Ala-Asp-
```

The amino acid sequence was determined in the same manner as adopted in regard to PLA-H4.

5) Amino acid composition:

Constant boiling point hydrochloric acid (0.2 ml) containing 0.1 % thioglycolic acid was added to TLA-H8 (0.5 $\mu$g), and the resultant mixture was hydrolyzed in a sealed tube at 110°C for 24 hours to determine the amino acid composition. The results are shown in Table 1.

TLA-H

1) Color and appearance: white powder.

2) water-solubility: easily soluble.

3) Molecular weight:

3-1) About 5,000 - 15,000 measured by gel filtration using Sephadex G-75. The marker proteins were bovine serum albumin (molecular weight, 67,000), egg albumin (molecular weight, 43,000), chymotripsinogen (molecular weight, 25,000) and ribonuclease A (molecular weight, 13,700) manufactured by Sigma, and the eluting buffer was 0.1M ammonium acetate (pH, 6.7).

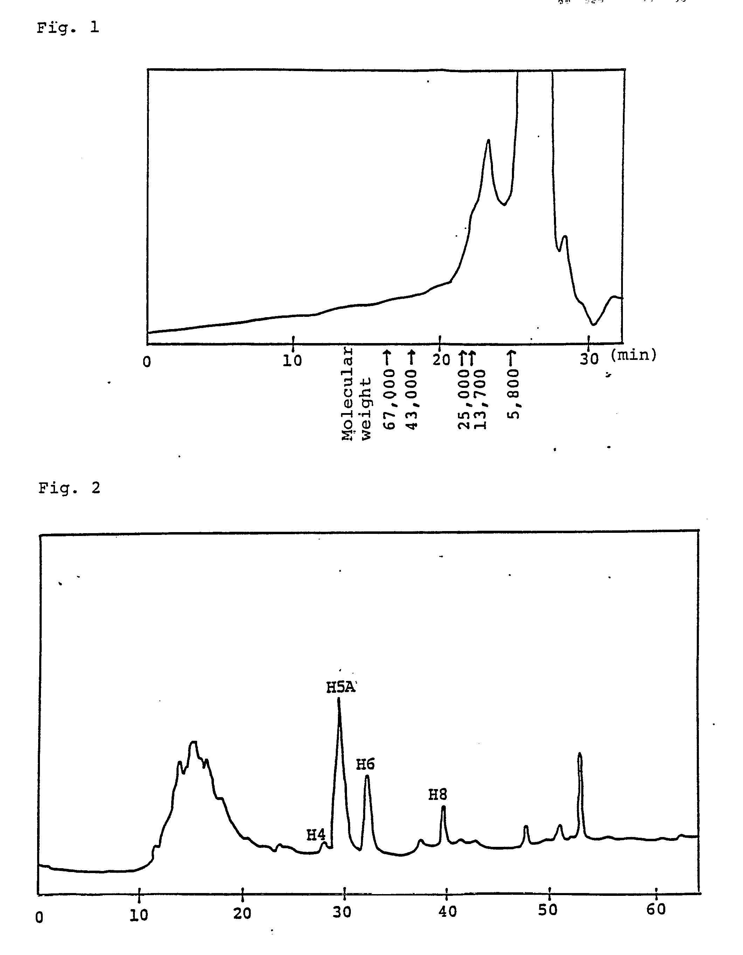

3-2) About 4,000 - 15,000 (cf. Fig. 1 of the accompanying drawing) measured by high speed gel filtration using TSK-G2000SW. The marker proteins were bovine serum albumin (molecular weight, 67,000), egg albumin (molecular weight, 43,000), ribonuclease A (molecular weight, 13,700) and pork insulin (molecular weight, 5,800) manufactured by Sigma, and the eluting buffer was M/15 phosphate buffer-M/10 sodium chloride (pH, 6.7).

4) Color reaction: positive in reactivity to Coomassie Brilliant Blue G-250 (confirmation for a protein).

Table 1

| Amino acid composition (mol %) | | | | | |
|---|---|---|---|---|---|
| | TLA-H4 | TLA-H5A | TLA-H5C | TLA-H6 | TLA-H8 |
| Asx | 10.8 | 10.7 | 10.4 | 10.8 | 12.3 |
| Thr | 6.0 | 8.9 | 8.4 | 8.9 | 4.7 |
| Ser | 8.3 | 3.9 | 4.6 | 4.1 | 6.1 |
| Glx | 14.7 | 16.1 | 16.4 | 16.1 | 12.6 |
| Pro | 2.7 | 4.0 | 4.0 | 4.0 | 3.1 |
| Cysteine | 0.2 | 0 | 0 | 0 | 2.3 |
| Gly | 13.1 | 6.0 | 7.1 | 6.3 | 9.8 |
| Ala | 5.9 | 3.0 | 4.0 | 3.1 | 9.4 |
| Val | 6.0 | 5.4 | 5.7 | 5.4 | 6.5 |
| Met | 1.4 | 1.4 | 1.4 | 1.4 | 1.8 |
| Ile | 5.8 | 8.9 | 8.2 | 8.9 | 4.5 |
| Leu | 11.5 | 12.3 | 11.4 | 12.3 | 6.7 |
| Tyr | 0.9 | 1.3 | 1.3 | 1.4 | 3.4 |
| Phe | 2.8 | 2.7 | 2.8 | 2.8 | 2.6 |
| Lys | 5.1 | 9.0 | 8.8 | 9.0 | 5.2 |
| His | 1.0 | 1.4 | 1.4 | 1.4 | 1.8 |
| Arg | 3.8 | 5.2 | 3.9 | 4.2 | 7.4 |

The anti-tumor proteins of the invention may be served in the form of biologically acceptable acid addition salts with organic acids (e.g. acetic acid, oxalic acid, maleic acid, succinic acid, fumaric acid) or inorganic acids (e.g. hydrochloric acid, hydrobromic acid, sulfuric acid). Such acid addition salts can be easily produced by a per se conventional procedure, for instance, dissolving the anti-tumor protein in water and adding a desired amount of an acid such as acetic acid.

The anti-tumor proteins according to the invention can be produced by various procedures, of which some typical procedures are explained below.

Procedure (i)

Extraction and purification of the anti-tumor protein from Tp strain:-

As the Tp strain, there may be used any one, and its typical example is the RH strain, tachyzoites of Toxoplasma gondii. The Tp strain as naturally available may be used as such, or it may be once proliferated in an animal body or by tissue culture cells, followed by isolation for the use.

The physical destruction of the Tp strain may be performed in a conventional method as described in JP-A-59-27832, for instance, by ultrasonic vibration and/or freezing-thawing. In case of ultrasonic vibration, the Tp strain is suspended in distilled water, physiological saline solution, phosphate buffer-sodium chloride solution (PBS), salt-equilibration Hanks' solution or the like, preferably distilled water, to make a concentration of about 100 mg/ml (2 x 10$^9$ Tp/ml) and subjected to ultrasonic vibration at a temperature of 4 to 15°C for a period of 1 to 5 minutes two or more times. In case of freezing-thawing, the Tp suspension prepared in the same manner as above is freezed at a temperature of -20 to -80°C and molten at a temperature of 20 to 37°C, and these freezing and melting are repeated several times, normally 5 times.

The thus obtained Tp destruction product is, if desired, subjected to elimination of insoluble materials by centrifugation (e.g. 16,000 G, 4°C, 60 minutes), a sodium chloride solution is added thereto for prevention of denaturation to make isotonic, and the resultant mixture is subjected to ultra-high speed centrifugation at 100,000 G or more (e.g. 4°C, 120 minutes) for recovery of the supernatant. The supernatant is then subjected to axenic filtration and freeze-dried to give the objective protein (i.e. Tp lytic antigen (hereinafter referred to as "TLA")) in a powdery state.

Purification of the powdery TLA as above obtained may be achieved by a per se conventional procedure such as gel filtration chromatography, ultrafiltration, dialysis and/or salting out. Preferably, the powdery TLA is dissovled in water or a physiological saline solution, and the resultant solution is subjected to molecular weight fractionation by gel filtration chromatography with a suitable filler such as Sephadex G-50, Sephadex G-100, Sephacryl S-200, Biogel P-30 or Biogel P-60, preferably Sephadex G-75. The fractions of 4,000 to 15,000 in molecular weight were collected and freeze-dried to give the proteinic substance, i.e. Tp lytic antigen-H (hereinafter referred to as "TLA-H"), as white powder. TLA-H thus obtained is a mixture of the anti-tumore proteins, which falls within the scope of the invention.

Isolation of any individual protein from TLA-H can be achieved by a per se conventional purification procedure such as high speed liquid chromatography, ion-exchange chromatography or affinity chromatography with a proper antibody. Preferably, TLA-H is dissolved in water or 0.1 % trifluoroacetic acid and purified by reverse phase liquid chromatography with a reverse phase filler such as a silica gel carrier conjugated with a $C_1$-$C_{18}$ alkyl group or a phenyl group. As the eluting solvent, there may be used acetonitrile, propanol, isopropanol or the like containing trifluoroacetic acid, acetic acid, formic acid, phosphoric acid or the like. For instance, TLA-H is fractionated by high speed liquid chromatography on RP-304 column (Bio-rad Inc.) (cf. Fig. 2 of the accompanying drawing), and the fraction eluted with an eluting solvent having an acetonitrile concentration of 30 to 35 % is freeze-dried to give individual proteinic substances such as TLA-H4, TLA-H5A, TLA-H5C, TLA-H6 and TLA-H8 as white powder, which are usually kept in a dark and cold place for storage.


Procedure (ii)


Chemical synthesis of the anti-tumor protein:-

The chemical synthesis of the antitumor protein of the invention may be accomplished by a synthetic method as conventionally adopted in the peptide chemistry. It may be a solid phase process or a liquid phase process. When the solid phase process is adopted, a C-terminal amino acid (protected at the amino group) is combined with an insoluble carrier through its carboxyl group, followed by elimination of the amino protecting group. Then, amino acids wherein the amino group is protected are stepwise combined thereto by condensation between the carboxyl group and the amino group. After the entire amino acid sequence is completed, the produced peptide is released from the insoluble carrier while elimination of the protecting group to obtain the objective anti-tumor protein.

In the above condensation, the reactive functional groups are preferred to be protected by conventional procedures. As the protective group for amino, there may be exemplified benzyloxycarbonyl, t-butyloxycarbonyl, p-methoxybenzyloxycarbonyl, 2-chlorobenzyloxycarbonyl, p-toluenesulfonyl, trifluoroacetyl, phthalyl, formyl, o-nitrophenylsulfonyl, 3-nitro-2-pyridinesulfenyl, diphenylphosphinothioyl, etc. Examples of the protective group for carboxyl are alkyl ester such as $C_1$-$C_4$ alkyl ester (e.g. methyl ester, ethyl ester, t-butyl ester), benzyl ester, p-nitrobenzyl ester, p-methylbenzyl ester, cyclohexyl ester, cyclopentyl ester, etc. As the protective group for guanido in Arg, there may be employed, for instance, p-toluenesulfonyl, mesitylene-2-sulfonyl, nitro, benzyloxycarbonyl, etc. Protection of a hydroxyl group in Ser, Thr or Tyr is not always necessary, and when it is to be protected, there may be used benzyl, 2,6-dichlorobenzyl, t-butyl, benzyloxycarbonyl, acetyl, etc. Likewise, Met may be protected in the form of sulfoxide, if necessary.

The condensation for peptide linkage formation may be carried out by a conventional process such as a process using a condensating agent, especially a carbodiimide type condensing agent (e.g. dicyclohexylcarbodiimide, 1-ethyl-3-(3-dimethylaminopropyl)carbodiimide), a symmetric acid anhydride process, a mixed acid anhydride process, an azide process, an active ester process, an oxidation-reduction process, a diphenylphosphorylazide process or a process with a carbodiimide type condensing agent and an additive (e.g. 1-hydroxybenzotriazole, N-hydroxysuccinimide).

For elimination of the protecting group, there may be also adopted a conventional process using trifluoroacetic acid, methanesulfonic acid, trifluoromethanesulfonic acid, hydrogen fluoride or liquid ammonia-sodium.

Purification of the protein thus produced may be effected according to a procedure as conventionally adopted in the peptide chemistry such as ion-exchange resin chromatography, partition chromatography, gel chromatography or reverse phase liquid chromatography.

Procedure (iii)

Enzymatic cleavage for obtaining the anti-tumor protein:-

The proteins as obtained in Procedure (i) or (ii) or ubiquitin similar thereto in chemical structure (cf. Goldstein, G. et al.: Proc.Natl.Acad.Sci., U.S.A., 72, 11-15 (1975); Harris Busch: Methods in Enzymol., 106, 238-262 (1984)) are treated with a suitable enzyme such as carboxypeptidase, trypsin, V8 protease, lysilendopeptidase, thermolysine, pepsin or pronase, preferably carboxypeptidase or lysilendopeptidase, for digestion, whereby an optional number of amino acids at the C-terminal position are eliminated to give the desired proteins.

The notable anti-tumor activity of the proteins according to the invention is well evidenced by the test results as set forth below.

Test 1

Sarcoma 180 (S-180) cells ($3 \times 10^6$) was transplanted subcutaneously to BALB/c mice (male, 5-week old) at their abdomen, 5 to 12 animals being grouped. The sarcoma was grown to a certain size about one week after the transplantation. The S-180 bearing mice were separated into two groups, of which the first one consisting of 12 animals was taken as the control group and the second one consisting of 5 animals was taken as the medicating group. The anti-tumor protein TLA-H (0.4 $\mu$g) obtained in Examples as hereinafter given was intramuscularly administered to each animal of the second group 3 times every week.

The growth of sarcoma was checked with lapse of tion by measurement of its volume [(long axis) x (short axis)$^2$ x 1/2] (mm$^3$), and the results are shown in Table 2 wherein the anti-tumor effect is indicated by the percentage of the growth rate in the medicating group (T) to that in the control (i.e. non-medicating) group (C). From such results, it is understood that TLA-H shows a prominent anti-tumor activity.

Table 2

| Antitumor effect of TLA-H (T/C) (%) | | | | | | | | | | | | |
|---|---|---|---|---|---|---|---|---|---|---|---|---|
| Protein | Dose ($\mu$g/mouse) | Numeber of test animal | Time elapsed after transplantation (day) | | | | | | | | | |
| | | | 7 | 9 | 12 | 15 | 19 | 22 | 26 | 29 | 33 | 36 |
| TLA-H | 0.4 | 5 | 100 | 65* | 50* | 46* | 47* | 40* | 51 | 56 | 58 | - |
| Note: | | | | | | | | | | | | |

* statistically significant ($p < 0.05$, t-test)

Test 2

In the same manner as in Test 1 but using the protein TLA-H4, TLA-H6 or TLA-H8 (0.18 $\mu$g) obtained in Examples as hereinafter given instead of TLA-H (0.4 $\mu$g), the test was carried out, and the results are shown in Table 3, from which it is understood that TLA-H4, TLA-H6 and TLA-H8 produce a remarkable anti-tumor effect.

Table 3

| Anti-tumor effect of TLA-H4, TLA-H6 and TLA-H8 (T/C) (%) | | | | | | | | | | | | | |
|---|---|---|---|---|---|---|---|---|---|---|---|---|---|
| Protein | Dose (μg/mouse) | Number of test animal | Time elapsed after transplantation (day) | | | | | | | | | | |
| | | | 7 | 9 | 12 | 15 | 19 | 22 | 26 | 29 | 33 | 36 |
| TLA-H4 | 0.18 | 5 | 100 | 77 | 60* | 51* | 53* | 45* | 65 | 59 | 54* | 82 |
| TLA-H6 | 0.18 | 6 | 100 | 80 | 55* | 46* | 46* | 40* | 59* | 61 | 62* | 77 |
| TLA-H8 | 0.18 | 6 | 100 | 84 | 63* | 59* | 74 | 82 | 109 | 91 | 95 | - |
| Note: | | | | | | | | | | | | | |
| * Statistically significant (p <0.05, t-test) | | | | | | | | | | | | | |

Test 3

The anti-tumor activity of the proteins (TLA-H5A, TLA-H6 and TLA-H6E) obtained in Examples as hereinafter given was compared with that of ubiquitin having a primary structure quite similar to that of said proteins.

In the same manner as in Test 1 but using the TLA-H5A (0.83 μg), TLA-H6 (0.17 μg), TLA-H6E (0.34 μg) or ubiquitin (0.17 μg or 5 μg) instead of TLA-H (0.4 μg), the test was carried out, and the results are shown in Table 4, from which it is understood that TLA-H5A, TLA-H6 and TLA-H6E produce a remarkable anti-tumor effect, while ubiquitin does not produce any material anti-tumor effect.

Table 4

| Anti-tumor effect of TLA-H5A, TLA-H6, TLA-H6E and ubiquitin (T/C) (%) | | | | | | | | | | | | | |
|---|---|---|---|---|---|---|---|---|---|---|---|---|---|
| Protein | Dose (μg/mouse) | Number of test animal | Time elapsed after transplantation (day) | | | | | | | | | | |
| | | | 7 | 11 | 14 | 18 | 21 | 25 | 28 | 32 | 35 | 39 |
| TLA-H5 | 0.83 | 6 | 100 | 79* | 68* | 61* | 65 | 69 | 75 | 86 | 69* | 71 |
| TLA-H6 | 0.17 | 6 | 100 | 69* | 64* | 53* | 54* | 55* | 58* | 64 | 55* | 51* |
| TLA-H6E | 0.34 | 6 | 100 | 74* | 68* | 63* | 67 | 66 | 73 | 70 | 58* | 62* |
| Ubiquitin | 0.17 | 6 | 100 | 85 | 95 | 104 | 101 | 106 | 106 | 107 | 94 | 89 |
| Ubiquitin | 5 | 6 | 100 | 94 | 92 | 91 | 96 | 103 | 94 | 103 | 95 | 87 |
| Note: | | | | | | | | | | | | | |
| * Statistically significant (p <0.05, t-test) | | | | | | | | | | | | | |

The anti-tumor proteins of the invention exert an anti-tumor activity to mammalian animals and are thus useful as anti-tumor agents. For instance, the proteins show a prominent anti-tumor activity against experimental tumors in mice or rats (e.g. transplanted Sarcoma 180, Meth A, methylcholanthrene-induced cancer). The term "anti-tumor activity" as herein used is intended to mean such a substantial degree of activity as can be practically employed as an anti-tumor agent. For instance, it covers the statistically significant activity (p < 0.05, t-test) in the test using Sarcoma 180 cell bearing mice.

The proteins of the invention are normally used in the form of aqueous solution or physiological saline solution for the purpose of inhibition of proliferation or control of metastasis of tumor cells. When desired, any adjuvant or additive as conventionally employed may be incorporated therein. Examples of the adjuvant and the additive are solubilizing agents, emulsifiers (e.g. mineral oil), buffers, pain-releasing agents, preservatives, coloring agents, etc.

Any particular limitation is not present on mammalian animals, to which the proteins of the invention may be administered. Thus, the proteins are applicable to various mammalian animals such as mouse, rat, dog, cattle, horse, goat, sheep, rabbit, pig, etc. in addition to human beings. Administration may be accomplished through ordinary routes such as intramuscular, subcutaneous, intracutaneous, abdominal and venal routes. The amount and time of the proteins to be given may be appropriately decided on the kind, bodyweight, age and symptom of the mammals as well as the purpose and route of the administration, and usually the proteins may be given with each dose of 1 to 5 mg/kg once to several times per day.

Practical embodiments for production of the anti-tumor proteins according to the invention are illustratively shown in the following Examples.

Example 1

Isolation of TLA-H4, TLA-H5A, TLA-H6 and TLA-H8:-

(1) Mice were infected with Tp RH strain, and on the 2nd day thereafter, their abdominal cavity was washed with Hanks' buffer, and the washing solution was centrifuged at 750G at 4°C for 10 minutes. The precipitate containing impurities such as abdominal cells was filtered with a nylon mesh column to remove such impurities. The Tp suspension thus obtained was centrifugally washed three times (750G, 4°C, 10 minutes) with HBSS solution. The precipitated Tp body was admixed with sterile distilled water to make a dilution of $2 \times 10^9$ Tp/ml, followed by stirring. The dilution was freezing-thawed three times in a freezer at -70°C and then subjected to ultrasonification in a ultrasonic vibrator Type W-220F (Wakenyaku K.K.) at 40 W for 1 minute five times for destruction of the Tp body. The resultant mixture was centrifuged at 16,000 G at 4°C for 60 minutes, and the supernatant was admixed with an equivalent amount of 1.7 % sodium chloride solution to make isotonic. The isotonic solution was then subjected to high speed centrifugation at 144,000 G at 4°C for 120 minutes, and the supernatant was further sterilized by filtration and dried in vacuo to give a proteinic substance TLA-144, which was kept in a dark and cold place for storage.

(2) TLA-144 (45 mg) was dissolved in water (2.3 ml), the resultant solution was added to a column ·(1.6 Ø x 96 cm) filled with Sephadex G-75 (Pharmacia) equilibrated with 0.1 M ammonium acetate buffer (pH, 4.8), and the column was eluted with the same buffer as above at a flow rate of 7 ml/hour. While monitoring the eluate at A280 nm, fractions (3.5 ml each) were obtained every 30 minutes. The fractions having a molecular weight of 4,000 to 15,000 were combined together and freeze-dried to give the proteinic substance TLA-H (about 0.2 mg) as white powder.

(3) TLA-H (about 0.2 mg) was charged in a column packed with a reverse phase filler "RP-304" (Biorad Inc.; 4.6 Ø x 250 mm) and equilibrated with 0.1 % trifluoroacetic acid, and the column was washed with 0.1 % trifluroacetic acid and eluted with increased concentrations of acetonitrile up to 57 % at a flow rate of 1.0 ml/min while monitoring the eluate at A220 nm. Fractions eluted with eluents having acetonitrile concentrations of 32.0 %, 32.5 %, 34.0 % and 38.0 % were collected and each repeatedly chromatographed to give TLA-H4 (0.01 mg), TLA-H5A (0.06 mg), TLA-H6 (0.05 mg) and TLA-H8 (0.01 mg) (cf. Fig. 2 of the accompanying drawing).

Example 2

Isolation of TLA-H4, TLA-H5A, TLA-H5C and TLA-H6:-

TLA-144 (93 mg) was dissolved in 0.85 % sodium chloride solution (2.3 ml), the resultant solution was added to a column (1.6 Ø x 96 cm) packed with a filler for gel filtration "Sephadex G-75" (Pharmacia) equilibrated with 0.1 M ammonium acetate buffer (pH, 4.8), and the column was eluted with the same buffer as above at a flow rate of 7.4 ml/hour. While monitoring the eluate at A280 nm, fractions (3.7 ml each) were obtained every 30 minutes. The fractions having a molecular weight of 5,000 to 15,000 were combined together and freeze-dried to give the proteinic substance TLA-H (about 0.74 mg) as white powder.

TLA-H (about 0.70 mg) as above obtained was charged in a column packed with a reverse phase filler "RP-304" (Biorad Inc.; 4.6 Ø x 250 mm) and equilibrated with 0.1 % trifluoroacetic acid, and the column was washed with 0.1 % trifluroacetic acid and eluted with increased concentrations of acetonitrile up to 57 % at a flow rate of 1.0 ml/min while monitoring the eluate at A220 nm. Fractions eluted with eluents having acetonitrile concentrations of 32.0 %, 32.5 %, 33.0 % and 34.0 % were collected and each repeatedly chromatographed to give TLA-H4 (0.01 mg), TLA-H5A (0.13 mg), TLA-H5C (0.07 mg) and TLA-H6 (0.04 mg).

Example 3

Analysis on the structure of TLA-H5A:-

TLA-H5A (30 μg) was dissolved in 0.05 M tris-hydrochloric acid (0.1 ml; pH, 9.2), lysyl endopeptidase C (1.5 μg) was added thereto, and incubation was carried out at 37° C for 15 hours. The fragmented peptides thus obtained were charged on a column (4.6 Ø x 250 mm) packed with a reverse phase filler "RP-304" and equilibrated with 0.1 % trifluoroacetic acid, followed by eluting with increased concentrations of acetonitrile up to 95 % at a flow rate of 1.0 ml/min while monitoring the eluate at A220 nm. Eight kinds of peptides eluted with eluents having acetonitrile concentrations of 0 to 40 % were hydrolyzed with 6N hydrochloric acid containing 0.1 % thioglycolic acid for 20 hours and subjected to analysis on amino acid composition using an amino acid analyzer "Hitachi 835-type" (Hitachi Seisakusho). Said peptides were also subjected to the Edman's degradation by the use of a 470A-type protein sequencer (Applied Biosystems Inc.), and the resultant phenylthiohydantoin-derived amino acid (PTH-amino acid) was analyzed by a 120A-type PTH analyzer (Applied Biosystem) for determination of the amino acid sequence.

Based on the results obtained in the analysis of the amino acid composition (cf. Table 1), the analysis of the N-terminal amino acid sequence and the analysis of the amino acid compositions and amino acid sequences of eight constituting peptides, the primary structure of TLA-H5A was determined as follows:

```
1                                        10
Met-Gln-Ile-Phe-Val-Lys-Thr-Leu-Thr-Gly-
                                         20
Lys-Thr-Ile-Thr-Leu-Asp-Val-Glu-Pro-Ser-
                                         30
Asp-Thr-Ile-Glu-Asn-Val-Lys-Ala-Lys-Ile-
                                         40
Gln-Asp-Lys-Glu-Gly-Ile-Pro-Pro-Asp-Gln-
                                         50
Gln-Arg-Leu-Ile-Phe-Ala-Gly-Lys-Gln-Leu-
                                         60
Glu-Asp-Gly-Arg-Thr-Leu-Ser-Asp-Tyr-Asn-
                                         70
Ile-Gln-Lys-Glu-Ser-Thr-Leu-His-Leu-Val-
                  74
Leu-Arg-Leu-Arg
```

Example 4

Analysis on the structure of TLA-H5C:-

TLA-H5C (20 ug) was dissolved in 0.05 M tris-hydrochloric acid (0.1 ml; pH, 9.2), lysyl endopeptidase C (1.0 μg) was added thereto, and incubation was carried out at 37° C for 15 hours. The fragmented peptides thus obtained were charged on a column (4.6 Ø x 250 mm) packed with a reverse phase filler "RP-304" and equilibrated with 0.1 % trifluoroacetic acid, followed by eluting with increased concentrations of acetonitrile up to 95 % at a flow rate of 1.0 ml/min while monitoring the eluate at A220 nm. Eight kinds of peptides eluted with eluents having acetonitrile concentrations of 0 to 40 % were hydrolyzed with 6N hydrochloric acid containing 0.1 % thioglycolic acid for 20 hours and subjected to analysis on amino acid composition using an amino acid analyzer "Hitachi 835-type" (Hitachi Seisakusho). Said peptides were also subjected to the Edman's degradation by the use of a 470A-type protein sequencer (Applied Biosystems Inc.), and the resultant phenylthiohydantoin-derived amino acid (PTH-amino acid) was analyzed by a 120A-type PTH analyzer (Applied Biosystem) for determination of the amino acid sequence.

Based on the results obtained in the analysis of the amino acid composition (cf. Table 1), the analysis of the N-terminal amino acid sequence and the analysis of the amino acid compositions and amino acid sequences of eight constituting peptides, the primary structure of TLA-H5C was determined as follows:

```
1                                        10
Met-Gln-Ile-Phe-Val-Lys-Thr-Leu-Thr-Gly-
                                         20
Lys-Thr-Ile-Thr-Leu- X -Val-Glu-Pro-Ser-
                                         30
Asp-Thr-Ile-Glu-Asn-Val-Lys-Ala-Lys-Ile-
                                         40
Gln-Asp-Lys-Glu-Gly-Ile-Pro-Pro-Asp-Gln-
                                         50
Gln-Arg-Leu-Ile-Phe-Ala-Gly-Lys-Gln-Leu-
                                         60
Glu-Asp-Gly-Arg-Thr-Leu-Ser-Asp-Tyr-Asn-
                                         70
Ile-Gln-Lys-Glu-Ser-Thr-Leu-His-Leu-Val-
              74
Leu-Arg-Leu-Glx
```

wherein X represents Asp or Glu.


Example 5


Analysis on the structure of TLA-H6:-

TLA-H6 (15 g) was dissolved in 0.05 M tris-hydrochloric acid (0.1 ml; pH, 9.2), lysyl endopeptidase C (0.75 μg) was added thereto, and incubation was carried out at 37°C for 15 hours. The fragmented peptides thus obtained were charged on a column (4.6 Ø x 250 mm) packed with a reverse phase filler "RP-304" and equilibrated with 0.1 % trifluoroacetic acid, followed by eluting with increased concentrations of acetonitrile up to 95 % at a flow rate of 1.0 ml/min while monitoring the eluate at A220 nm. Eight kinds of peptides eluted with eluents having acetonitrile concentrations of 0 to 40 % were hydrolyzed with 6N hydrochloric acid containing 0.1 % thioglycolic acid for 20 hours and subjected to analysis on amino acid composition using an amino acid analyzer "Hitachi 835-type" (Hitachi Seisakusho). Said peptides were also subjected to the Edman's degradation by the use of a 470A-type protein sequencer (Applied Biosystems Inc.), and the resultant phenylthiohydantoin-derived amino acid (PTH-amino acid) was analyzed by a 120A-type PTH analyzer (Applied Biosystem) for determination of the amino acid sequence.

Based on the results obtained in the analysis of the amino acid composition (cf. Table 1), the analysis of the N-terminal amino acid sequence and the analysis of the amino acid compositions and amino acid sequences of eight constituting peptides, the primary structure of TLA-H6 was determined as follows:

```
1                                            10
Met-Gln-Ile-Phe-Val-Lys-Thr-Leu-Thr-Gly-
                                             20
Lys-Thr-Ile-Thr-Leu- X -Val-Glu-Pro-Ser-
                                             30
Asp-Thr-Ile-Glu-Asn-Val-Lys-Ala-Lys-Ile-
                                             40
Gln-Asp-Lys-Glu-Gly-Ile-Pro-Pro-Asp-Gln-
                                             50
Gln-Arg-Leu-Ile-Phe-Ala-Gly-Lys-Gln-Leu-
                                             60
Glu-Asp-Gly-Arg-Thr-Leu-Ser-Asp-Tyr-Asn-
                                             70
Ile-Gln-Lys-Glu-Ser-Thr-Leu-His-Leu-Val-
                73
Leu-Arg-Leu
```

wherein X represents Asp or Glu.

Example 6

Synthesis of TLA-H6D:-

As the resin, there was used chloromethylated polystyrenevinylbenzene resin (crosslinked with 1 % divinylbenzene and containing 0.64 mmol chloride/1 g) of 100 to 200 mesh in particle size.

Boc-Leu-OH (21 mmol) was dissolved in a mixture of ethanol (20 ml) and water (6 ml), and the resultant mixture was adjusted to pH 7 with aqueous cesium carbonate, concentrated under reduced pressure and dried. Dimethylformamide (126 ml) was added thereto, and the resultant mixture was further combined with the chloromethylated resin (12.8 mmol), and esterification was effected at 50° C for 24 hours. The thus prepared amino acid-combined resin was collected by filtration, washed with 90 % dimethylformamide, dimethylformamide and ethanol in order and dried. The amino acid-combined resin (6 g) was admitted in a solid phase reactor and allowed to coupling stepwise with each of the reagents as set forth below in the presence of an equivalent amount of dicyclohexylcarbodiimide according to Schedule 1.

Reagents:-

Boc-Arg(Tos)-OH, Boc-Leu-OH, Boc-Val-OH, Boc-Leu-OH, Boc-His(Tos)-OH, Boc-Leu-OH;
Boc-Thr(Bzl)-OH, Boc-Ser(Bzl)-OH, Boc-Glu(OcHex)-OH, Boc-Lys(Cl-Z)-OH, Boc-Gln-OH, Boc-Ile-OH, Boc-Asn-OH, Boc-Tyr(Cl-z-Bzl)-OH, Boc-Asp(OcHex)-OH;
Boc-Ser(Bzl)-OH, Boc-Leu-OH, Boc-Thr(Bzl)-OH, Boc-Arg(Tos)-OH;
Boc-Gly-OH, Boc-Asp(OcHex)-OH, Boc-Glu(OcHex)-OH, Boc-Leu-OH;
Boc-Gln-OH, Boc-Lys(Cl-Z)-OH, Boc-Gly-OH, Boc-Ala-OH, Boc-Phe-OH, Boc-Ile-OH;
Boc-Leu-Oh, Boc-Arg(Tos)-OH, Boc-Gln-OH, Boc-Gln-OH, Boc-Asp(OcHex)-OH, Boc-Pro-OH, Boc-Pro-OH, Boc-Ile-OH, Boc-Gly-OH, Boc-Glu(OcHex)-OH;
Boc-Lys(Cl-Z)-OH, Boc-Asp(OcHex)-OH, Boc-Gln-OH, Boc-Ile-OH, Boc-Lys(Cl-Z)-OH;
Boc-Ala-OH, Boc-Lys(Cl-Z)-OH, Boc-Val-OH, Boc-Asn-OH, Boc-Glu(OcHex)-OH;
Boc-Ile-OH, Boc-Thr(Bzl)-OH, Boc-Asp(OcHex)-OH, Boc-Ser(Bzl)-OH;
Boc-Pro-OH, Boc-Glu(OcHex)-OH, Boc-Val-OH, Boc-Asp(OcHex)-OH;
Boc-Leu-OH, Boc-Thr(Bzl)-OH, Boc-Ile-OH, Boc-Thr(Bzl)-OH, Boc-Lys(Cl-Z)-OH;
Boc-Gly-OH, Boc-Thr(Bzl)-OH, Boc-Leu-OH, Boc-Thr(Bzl)-OH, Boc-Lys(Cl-Z)-OH; and
Boc-Val-OH, Boc-Phe-OH, Boc-Ile-OH, Boc-Gln-OH, Boc-Met-OH.

EP 0 341 734 A2

Schedule 1

| Step | Treatment | Treating liquid | Time (min) x Frequency |
|------|-----------|-----------------|------------------------|
| 1 | Washing | Methylene chloride (60 ml) | 2 x 3 |
| 2 | Elimination of protected group | 50 % TFA – 5 % ethanediol – 45 % methylene chloride (v/v) (60 ml) | 3 x 1 |
| 3 | Washing | Methylene chloride (60 ml) | 2 x 4 |
| 4 | Washing | Methanol (60 ml) | 2 x 2 |
| 5 | Neutralization | 10 % Triethylamine – 90 % methylene chloride (v/v) (60 ml) | 1 x 2 |
| 6 | Washing | Methanol (60 ml) | 2 x 2 |
| 7 | Neutralization | 10 % Triethylamine – 90 % methylene chloride (v/v) (60 ml) | 1 x 2 |
| 8 | Washing | Methanol (60 ml) | 2 x 2 |
| 9 | Washing | Methylene chloride (60 ml) | 2 x 3 |
| 10 | Coupling | Each Boc–amino acid (6 mmol) and DCC (6 mmol) in methylene chloride (30 ml) | 120 x 1 |
| 11 | Washing | Methylene chloride (60 ml) | 2 x 1 |
| 12 | Washing | 50 % DMF – 50 % methylene chloride (v/v) (60 ml) | 2 x 2 |
| 13 | Neutralization | 10 % Triethylamine – 90 % methylene chloride (v/v) (60 ml) | 1 x 1 |

EP 0 341 734 A2

| Step | Treatment | Treating liquid | Time (min) x Frequency |
|------|-----------|-----------------|------------------------|
| 14 | Washing | Methanol (60 ml) | 2 x 2 |
| 15 | Washing | Methylene chloride (60 ml) | 2 x 2 |
| 16 | Acetylation | 25 % Acetic anhydride – 50 % methylene chloride (v/v) (60 ml) | 20 x 1 |
| 17 | Washing | Methylene chloride (60 ml) | 2 x 2 |
| 18 | Washing | Methanol (60 ml) | 2 x 2 |
| 19 | Washing | Methylene chloride (60 ml) | 2 x 2 |

After each coupling, a small amount of the resin was subjected to testing with ninhydrin; in case of a positive result (blue coloring), the reaction was taken as incomplete, and couping was repeated using the same protected amino acid as previously employed with addition of N-hydroxybenzotriazole or p-nitrophenol, whereby the intermediate TLA-H6D peptide resin was obtained.

To the TLA-H6D peptide resin thus obtained (0.5 g), anisole (3 ml) and anhydrous hydrogen fluoride (20 ml) were added, and the resultant mixture was allowed to react at -20°C for 60 minutes and at 0°C for 60 minutes, followed by concentration under reduced pressure. Diethyl ether (300 ml) was added thereto, and the mixture was stirred for 30 minutes, filtered, washed with diethyl ether (200 ml) and extracted with 5 % acetic acid (200 ml). The precipitated resin was collected by filtration and washed with water (100 ml). The filtrate and the washing solution were combined together and purified by reverse phase column chromatography (YMC-AM324ODS, 1 cm x 25 cm; RP-304 (Bio-rad), 4.6 mm x 25 cm) twice to obtain a polypeptide. By HPLC analysis, this polypeptide was identified with TLA-H6 as obtained in Example 1. On the amino acid analysis, the values as found showed compliance with those as calculated.

Amino acid analysis: hydrolyzed with 6N hydrochloric acid-1 % phenol at 110°C for 24 hours; analyzed by PICO-TAG (reverse phase-PTC amino acid). The following results were obtained, in which the parentesized values indicate the theoretical ones: Asp, 7.6 (8); Glu, 11.4 Ser, 2.9 (3); Gly, 4.3 (4); His, 0.9 (1), Arg, 3.0 (3); Thr, 6.5 (7); Ala, 2.1 (2), Pro, 3.1 (3); Thy, 1.0 (1); Val, 3.8 (4); Met, 0.9 (1); Ile, 6.9 (7); * Leu, 9.0 (9); Phe, 1.8 (2); Lys, 6.8 (7).

The reaction was generally conducted accoradging to said Schedule 1. However, the coupling with Boc-Gln-OH and Boc-Asn-OH at Step 10 was performed with addition of an additive, and the coupling with Boc-Arg(Tos)-OH, Boc-Leu-OH, Boc-Ile-OH, Boc-Asn-OH and Boc-Gln-OH was carried out in the presence of 30 % DMF-70 % methylene chloride (v/v) instead of methylene chloride. Further, the second and subsequent couplings were performed in a mixture of DMF, dimethylsulfoxide and 1-methyl-2-pyrrodinone or methylene chloride instead of methylene chloride, and the reaction time was extended up to 10 hours at maximum.

## Example 7

To a solution of ubiquitin (Sigma) (5 mg) in 0.05 M acetic acid buffer (0.8 ml), carboxypeptidase P (17 μg) was added, and the resultant mixture was incubated at 37°C for 15 hours. The reaction mixture was charged on a column packed with a reverse phase filler "RP-304" (4.6 Ø x 250 mm) and equilibrated with 0.1 % trifluoroacetic acid, and then elution with eluents having increased concentrations of acetonitrile up to 43 % at a flow rate of 1.0 ml/min while monitoring the eluate at A220 nm. Fractions eluted with eluents having acetonitrile concentrations of 33 to 34 % were collected and purified on the same column as above to give TLA-H6E (2.6 mg).

By the amino acid analysis, TLA-H6 was identified with the derivative of ubiquitin lacking three amino acid residues at the C-terminal position.

Amino acid analysis (mol %): Asx, 7.2 (7); Thr, 6.8 (7), Ser, 2.7 (3), Glx, 13.0 (12); Pro, 3.2 (3); Gly, 4.0 (4), Ala, 2.0 (2); Val, 4.0 (4); Met, 1.0 (1); Ile, 7.0 (7); Leu, 9.3 (9); Tyr, 1.0 (1); Phe, 2.0 (2); Lys, 7.3 (7); His, 1.0 (1) and Arg, 3.1 (3).

## Claims

1. A physiologically active protein which has a molecular weight (determined by gel filtration with Sephadex G-75) of 4,000 to 15,000, is obtainable from the water-soluble fraction of protozoan Toxoplasma and shows an anti-tumor activity against sarcoma transplanted on mouse.

2. A process for purification of the protein according to claim 1 by the use of reverse phase liquid chromatography.

3. An anti-tumor agent which comprises as the active ingredient the protein according to claim 1.

*) standard amino acid.

Fig. 1

Molecular weight
67,000
43,000
25,000
13,700
5,800

Fig. 2

H5A

H6

H8

H4